# EUROPEAN PATENT SPECIFICATION

(11) **EP 3 564 214 B1**
(45) Date of publication and mention of the grant of the patent: **03.07.2024**
(21) Application number: 18170728.2
(22) Date of filing: 04.05.2018
(51) Int. Cl.: C07D 231/56, A61P 25/00, A61K 31/416

(54) **INDAZOLE DERIVATIVES AS MODULATORS OF THE CANNABINOID SYSTEM**
INDAZOLDERIVATE ALS MODULATOREN DES CANNABINOIDSYSTEMS
DÉRIVÉS D'INDAZOLE EN TANT QUE MODULATEURS DU SYSTÈME DE CANNABINOÏDES

(43) Date of publication of application: 06.11.2019
(73) Proprietor: Universita' Degli Studi G. D'annunzio Chieti - Pescara, 66100 Chieti (IT); Biological Research Centre, 6726 Szeged (HU)
(72) Inventor: TÖMBÖLY, Csaba, 6772 DESZK (HU); DVORÁCSKÓ, Szabolcs, 6725 SZEGED (HU); MOLLICA, Adriano, 66100 CHIETI (IT); MACEDONIO, Giorgia, 66100 CHIETI (IT); STEFANUCCI, Azzurra, 66100 CHIETI (IT)
(74) Representative: Currado, Luisa

(56) References cited:
- WO-A1-2009/106982
- MILANO WALTER ET AL: "The role of endocannabinoids in the control of eating disorders", DISEASES AND DISORDERS, vol. 1, no. 1, 13 November 2017 (2017-11-13), XP055814884, DOI: 10.15761/JDD.1000103 Retrieved from the Internet: URL:https://www.oatext.com/pdf/JDD-1-103.p df>
- Scherma Maria ET AL: "Cannabinoid CB 1 /CB 2 receptor agonists attenuate hyperactivity and body weight loss in a rat model of activity-based anorexia : Endocannabinoid system and activity-based anorexia", BRITISH JOURNAL OF PHARMACOLOGY, vol. 174, no. 16, 11 July 2017 (2017-07-11), pages 2682-2695, XP055814885, UK ISSN: 0007-1188, DOI: 10.1111/bph.13892 Retrieved from the Internet: URL:https://api.wiley.com/onlinelibrary/td m/v1/articles/10.1111%2Fbph.13892>

## Description

### Field of the invention

The present invention refers to the chemical field, in particular to indazole derivatives as modulators of the cannabinoid system, s which are useful in the treatment of obesity, eating diseases, cancer.

### Background of the invention

Lonidamine is a derivative of indazole-3-carboxylic acid, known as antispermatogenic agent and as male contraceptive. It is also an anti-tumor drug available for cancer treatment or benign prostatic hyperplasia, adjuvant to radiotherapy, chemotherapy, photodynamic therapy and hyperthermia in the treatment of advanced tumors such as lung carcinoma, breast, kidney, prostate, brain, colorectal and liver metastases (Floridi, A.; Paggi, M. G.; D'Atri, S.; De Martino, C.; Marcante, M. L.; Silvetrini, B.; Caputo, A. Effect of Lonidamine on the energy metabolism of ehrlich ascites tumor cells. Cancer Research 1981, 41, 4661-4666; Floridi, A. and Lehninger, A. L. Action of the antitumor and antispermatogenic agent lonidamine on electron transport in ehrlich ascites tumor mitochondria. Archives Biochem. Biophys. 1983, 226, 73-83; Caputo, A. and Silvestrini, B. Lonidamine a new approach to cancer therapy. Oncology 1984, 41, 2-6). Lonidamine blocks the rate of oxygen consumption in neoplastic and normal cells inducing an aerobic lactate production by normal cells only (Floridi, A.; Delpino, A.; Nista, A.; Feriozzi, R.; Marcante, M. L.; Silvestrini, B.; Caputo, A. Effect of lonidamine on protein synthesis in neoplastic cells. Exp. Molecular Pathology 1985, 42, 293-305; Roehrborn, C. G. The development of lonidamine for benign prostatic hyperplasia and other indications. Review in urology 2005, 7, S12-S20). Lonidamine was approved in Italy for oncology indications, with mild and temporary side effects and the combination of standard antineoplastic agents has been widely studied for the treatment of solid tumors (Uchiyama, N.; Matsuda, S.; Kawamura, M.; Kikura-Hanajiri, R.; Goda, Y. Two new type cannabimimetic quinolinyl carboxylates, QUPIC and QUCHIC, two new cannabimimetic carboxamide derivatives, ADB-FUBINACA and ADBICA, and five synthetic cannabinoids detected with a thiophene derivative a-PVT and an opioid receptor agonist AH-7921 identified in illegal products. Forensic toxicol. 2013, 10.1007/s11419-013-0182-9; Banister, S. D.; Moir, M.; Stuart, J.; Kevin, R. C.; Wood, K. E.; Longworth, M.; Wilkinson, S. M.; Beinat, C.; Buchanan, A. S.; Glass, M.; Connor, M.; McGregor, I. S.; Kassiou, M. Pharmacology of indole and indazole synthetic cannabinoid designer drugs AB-FUBINACA, ADB-FUBINACA, AB-PINACA, ADB-PINACA, 5F-AB-PINACA, 5F-ADB-PINACA, ADBICA, and 5F-ADBICA. ACS Chem Neurosci. 2015, 6, 1546-1559). Lonidamine is a potent enhancer of anti-cancer agents, able to replace the traditional chemotherapy, but has a limited solubility at neutral pH, thus more soluble derivatives administered by intravenous route are necessary to control dosing schedules (Nancolas, B.; Guo, L.; Zhou, R.; Nath, K.; Nelson, D. S.; Leeper, D. B.; Blair, !. A.; Glickson, J. D.; Halestrap, A. P. The anti-tumor agent lonidamine is a potent inhibitor of the mithocondrial pyruvate carrier and plasma membrane monocarboxylate transporters. Biochem. J. 2016, 473, 929-936; Nath, K.; Guo, L.; Nancolas, B.; Nelson, D. S.; Shestov, A. A.; Lee, S.-C.; Roman, J.; Zhou, R.; Leeper, D. B.; Halestrap, A. P.; Blair, I. A.; Glickson, J. D. Mechanism of antineoplastic activity of lonidamine. Biochim. Biophys. Acta 2016, 1866, 151-162).

The structure of Lonidamine is similar to the cannabimimetic carboxamide derivatives, FUBINACAs.

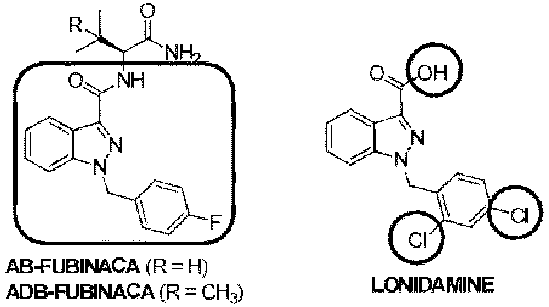

The cannabimimetic carboxamide derivative ADB-FUBINACA has been reported to show a potent affinity for CB1 cannabinoid receptor. This synthetic cannabinoid (SC) has an indazole scaffold joined to the amino acid derivative L-tert-leucinamide; its parent compound AB-FUBINACA possesses the same indazole scaffold but featuring an L-valinamide residue. Preliminary SAR investigations suggest that L-tert-leucinamide derivatives have greater potency in vitro at CB1 receptors than the L-valinamide analogues. AB-FUBINACA is able to decrease body temperature and heart rate dose-dependently, indicating that this SC behaves as a cannabimimetic in vivo. The synthetic cannabinoid (SC) has an indazole scaffold joined to the amino acid derivative L-tert-leucinamide; its parent compound AB-FUBINACA possesses the same indazole scaffold but featuring an L-valinamide residue. Preliminary SAR investigations suggest that L-tert-leucinamide derivatives have greater potency in vitro at CB1 receptors than the L-valinamide analogues (Roehrborn, C. G. The development of lonidamine for benign prostatic hyperplasia and other indications. Review in urology 2005, 7, S12-S20; Uchiyama, N.; Matsuda, S.; Kawamura, M.; Kikura-Hanajiri, R.; Goda, Y. Two new type cannabimimetic quinolinyl carboxylates, QUPIC and QUCHIC, two new cannabimimetic carboxamide derivatives, ADB-FUBINACA and ADBICA, and five synthetic cannabinoids detected with a thiophene derivative a-PVT and an opioid receptor agonist AH-7921 identified in illegal products. Forensic toxicol. 2013, 10.1007/s11419-013-0182-9; Banister, S. D.; Moir, M.; Stuart, J.; Kevin, R. C.; Wood, K. E.; Longworth, M.; Wilkinson, S. M.; Beinat, C.; Buchanan, A. S.; Glass, M.; Connor, M.; McGregor, I. S.; Kassiou, M. Pharmacology of indole and indazole synthetic cannabinoid designer drugs AB-FUBINACA, ADB-FUBINACA, AB-PINACA, ADB-PINACA, 5F-AB-PINACA, 5F-ADB-PINACA, ADBICA, and 5F-ADBICA. ACS Chem Neurosci. 2015, 6, 1546-1559; Nancolas, B.; Guo, L.; Zhou, R.; Nath, K.; Nelson, D. S.; Leeper, D. B.; Blair, I. A.; Glickson, J. D.; Halestrap, A. P. The anti-tumor agent lonidamine is a potent inhibitor of the mithocondrial pyruvate carrier and plasma membrane monocarboxylate transporters. Biochem. J. 2016, 473, 929-936).

EP2502624 discloses lonidamine analogues used in male contraception and cancer treatment. WO2006/015263 discloses lonidamine analogs for cancer treatment and in particular benign prostatic hyperplasia. WO2006/015191 discloses multicyclic lonidamine analogs for cancer treatment and in particular benign prostatic hyperplasia. WO2011/005759 discloses lonidamine analogs for fertility management. US2009/0197911 discloses lonidamine analogs for the treatment of polycystic kidney disease. WO2006/015283 and WO2004/064735 and US2007/00115771 and US2007/0043057 disclose lonidamine analogs for the treatment of benign prostatic hyperplasia. WO2006/010077 discloses lonidamine analogs and prodrugs for the treatment of benign prostatic hyperplasia.

WO2009/106982 discloses indazole derivatives and their use as for the treatment of conditions related to cannabinoid receptor.

Scherma M. et al., Cannabinoid CB1 /CB2 receptor agonists attenuate hyperactivity and body weight loss in a rat model of activity-based anorexia, Br J Pharmacol., 2017 Aug;174(16):2682-2695. doi: 10.1111/bph.13892. Epub 2017 Jul 11 discloses that subchronic treatment of ABA rodents with Δ9-tetrahydrocannabinol and compound CP-55,940 reduce body loss and running wheel activity, thus being useful for treating Anorexia nervosa (AN).

Milano W. et al. The role of endocannabinoids in the control of eating disorders Diseases and Disorders, 1(!) 13 november 2017, discloses that CB1 receptor modulators can be used for the treatment of eating disorders (ED).

### Technical problem

In view of the findings of the prior art the same inventors designed and synthesized a series of indazole derivatives based on Lonidamine and containing amino acid residues with the aim to improving their activity on the cannabinoid receptors (CB receptors). The compounds of the present invention differ from other cannabinoids with indazole scaffold known in the art for the ester or acidic terminus on the indazole scaffold and for the presence of substituents not being fluorine on the aromatic ring, which act in modulating the potency and the efficacy of the compound. In particular, the derivatization at the C-terminus exerts a modulatory effect on the central nervous system (CNS) by allowing to shift the effect of the drugs, from full agonist to partial agonist or to antagonist. The same inventors also verified that in comparison with lonidamine, the addition of aminoacidic residues on the carboxy terminus on the lonidamine scaffold, which doesn't have any affinity for cannabinoid receptor, it gives rise to potent cannabinoid modulators. The technical problem is solved by providing compounds exhibiting a broad range of activities, such as affinity for CB receptors, stimulation of G-proteins, selectivity for different CB receptors which are useful in the treatment of pain, obesity, eating diseases, nausea, cancer and avoiding the side effects in AIDS patients.

### Object of the invention

The above technical problem is solved by providing the compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid (*S*)-methyl 2-(1-(2,4-dichlorobenzyl)-*1H*-indazole-3-carboxamido)-4-methylpentanoate

The compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate as a medicament.

The compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-1H-indazole-3-carboxamido)-4-methylpentanoic acid
(*S*)-methyl 2-(1-(2,4-dichlorobenzyl)-*1H*-indazole-3-carboxamido)-4-methylpentanoate are used as modulator of cannabinoids receptors.

The compounds selected form the group consisting of:
(*S*)-2-(2,4-dichlorobenzyl)-*1H*-indazole-3-carboxamido)-3,3-dimethylbutanoic acid
(S)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(S)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate are used as modulator of cannabinoids receptors.

The compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate are used as antagonist of cannabinoids receptors.

The compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate are used inverse agonist of cannabinoids receptors.

The compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate are used as anorectic agent.

Another object of the present invention is compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate for the treatment of eating diseases.

Another object of the present invention is compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate

in combination with at least one chemoterapeutic agent for the treatment of cancer.

Further characteristic of the present invention will be clarified by the following detailed description, with reference to the claims and the drawings.

### Brief description of the drawings

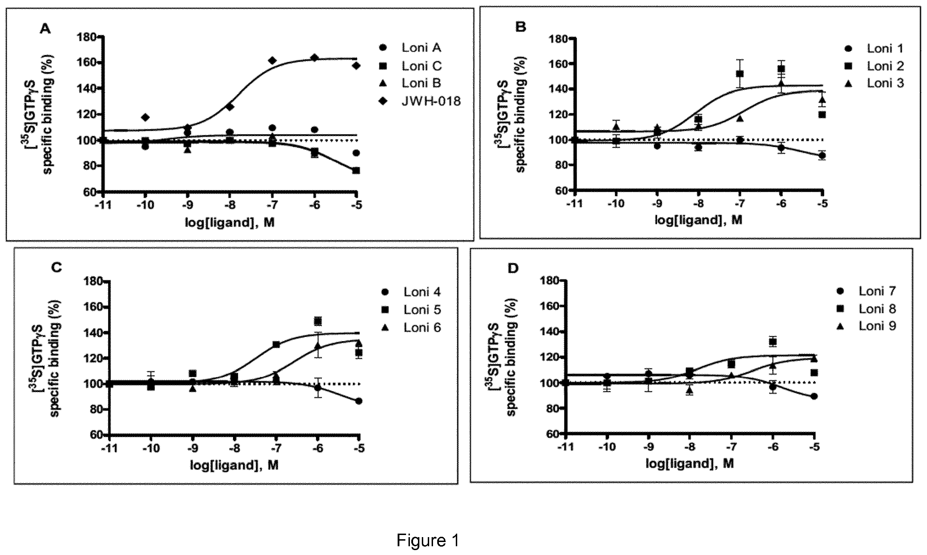

Figure 1 shows the cannabinoid receptor binding affinity of LONI A-C and LONI 1-9 ligands in [³H]WIN55,212-2 competition binding assays to rat whole brain membrane homogenates. Figures represent the specific binding of the radioligand in percentage in the presence of increasing concentrations (10⁻¹⁰-10⁻⁵ M) of the indicated ligands. "Total" on the x-axis indicates the total specific binding of the radioligand, which is measured in the absence of the unlabeled compounds. Data are expressed as percentage of mean specific binding ± S.E.M. (n≥ 3).

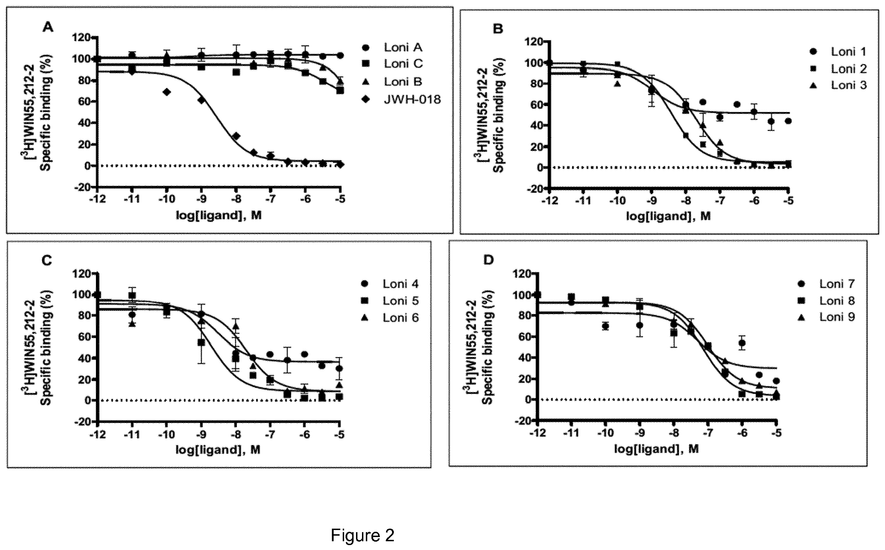

Figure 2 shows the G-protein stimulatory effects of LONI A-C and LONI 1-9 ligands in [³⁵S]GTPγS binding assays in rat brain membrane homogenates. Figures represent relative specific binding of [³⁵S]GTPγS in the presence of increasing concentrations (10⁻¹¹-10⁻⁵ M) of the indicated compounds. Data are expressed as percentage of mean specific binding ± S.E.M. (n≥ 3).

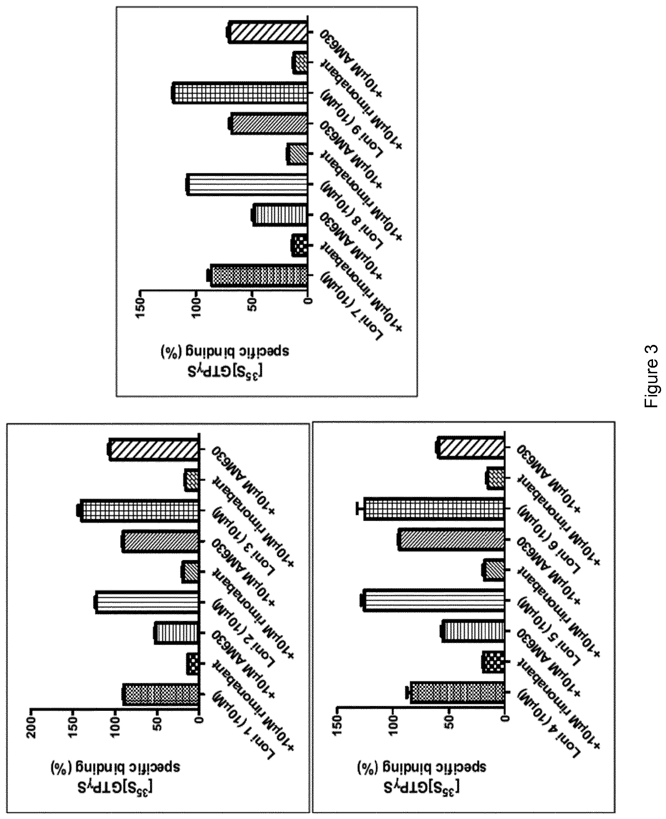

Figure 3 shows the CB1/CB2 cannabinoid receptors selectivity of LONI A-C and LONI 1-9 ligands in [³⁵S]GTPγS binding assays in rat brain membrane homogenates. The specific binding of [³⁵S]GTPγS is represented in percentage in the presence or absence of 10 µM rimonabant (CB1 antagonist/inverse agonist) or AM630 (CB2 antagonist/inverse agonist). Data are expressed as percentage of mean specific binding ± S.E.M. (n≥ 3).

### Detailed description of the invention

### Definitions

Within the meaning of the present invention modulator means a compound acting as an activator, an inhibitor, or both.

Within the meaning of the present invention agonist means a compound able to bind a receptor and to produce a biological response.

Within the meaning of the present invention antagonist means a compound able to bind a receptor and to reverse the effects of agonists.

Within the meaning of the present invention inverse agonist meas an agent that binds to the same receptor as an agonist but induces a pharmacological response opposite to that agonist.

Within the meaning of the present invention cannabinoid receptors are a class of cell membrane receptors of the G protein-coupled receptor superfamily, being part of the endocannabinoid system, involved in a variety of physiological processes such as appetite, pain-sensation, mood, and memory. Cannabinoid receptors are classified in CB1 and CB2, CB1 receptor is expressed mainly in central nervous system, lungs, liver and kidneys. The CB2 receptor is expressed mainly in the immune system and in hematopoietic cells.

Within the meaning of the present invention an anorectic agent is a compound which reduces appetite and can be used in helping weight loss.

Within the meaning of the present invention the side effects related to AIDS are chemotherapy-related nausea and vomiting, loss of appetite and loss of weight.

Within the meaning of the present invention eating dosorders means disorders defined by abnormal eating habits, such as binge eating, anorexia nervosa, bulimia nervosa, pica, rumination disorder, avoidant/restrictive food intake disorder.

Within the meaning of the present invention, chemotherapeutic agents or cytotoxic agents are compounds used in chemotherapy for cancer such as actinomycin, all-trans retinoic acid, azacitidine, azathioprine, bleomycin, bortezomib, carboplatin, capecitabine, cisplatin, chlorambucil, cyclophosphamide, cytarabine, daunorubicin, docetaxel, doxifluridine, doxorubicin, epirubicin, epothilone, etoposide, fluorouracil, gemcitabine, hydroxyurea, idarubicin, imatinib, irinotecan, mechlorethamine, mercaptopurine, methotrexate, mitoxantrone, oxaliplatin, paclitaxel, pemetrexed, teniposide, tioguanine, topotecan, valrubicin, vemurafenib, vinblastine, vincristine, vindesine.

Within the meaning of the present invention an antiemetic is a compound being effective against vomiting and nausea.

Within the meaning of the present invention an analgesic is a compound being used to achieve analgesia, relief from pain.

The present invention provides the compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-1H-indazole-3-carboxamido)-4-methylpentanoic acid
(*S*)-methyl 2-(1-(2,4-dichlorobenzyl)-*1H*-indazole-3-carboxamido)-4-methylpentanoate

Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate
for use as medicaments.

Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate
as modulator of cannabinoids receptors.

Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate
as agonist of cannabinoids receptors.

Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate
as antagonist of cannabinoids receptors.

Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate
as inverse agonist of cannabinoids receptors.

Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate
as anorectic agent.

Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate
for the treatment of eating diseases.

Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate

In combination with at least one chemotherapeutic agent for the treatment of cancer.

Chemotherapeutic agent is selected from the group consisting of actinomycin, all-trans retinoic acid, azacitidine, azathioprine, bleomycin, bortezomib, carboplatin, capecitabine, cisplatin, chlorambucil, cyclophosphamide, cytarabine, daunorubicin, docetaxel, doxifluridine, doxorubicin, epirubicin, epothilone, etoposide, fluorouracil, gemcitabine, hydroxyurea, idarubicin, imatinib, irinotecan, mechlorethamine, mercaptopurine, methotrexate, mitoxantrone, oxaliplatin, paclitaxel, pemetrexed, teniposide, tioguanine, topotecan, valrubicin, vemurafenib, vinblastine, vincristine, vindesine.

### Examples

### Material and methods

Lonidamine, solvents, reagents and the following amino acids Boc-*tert*-Leu-OH, Boc-Val-OH, Boc-Leu-OH, are commercially available and were purchased from Sigma-Aldrich (Milano, Italy). The structure of the intermediates and the final compounds were confirmed by ¹H-NMR spectra recorded on a 300MHz Varian Inova spectrometer (Varian Inc., Palo Alto, CA). Chemical shifts are reported in parts per million (δ) downfield from the internal standard tetramethylsilane (Me₄Si). The assignment of exchangeable protons was confirmed by the addition of D₂O. Homogeneity was confirmed by TLC on silica gel Merck 60 F254 (Merck, Darmstadt, Germany). Purifications via column chromatography, if required, were performed on Merck 60, 70-230 mesh ASTM silica gel column. Where mixtures of solvents are specified, the ratios are given as volume/volume. Unless otherwise indicated, all aqueous solutions were saturated. All reactions involving air- or moisture-sensitive compounds were performed under a nitrogen atmosphere using dried glassware and syringes. Analytical thin-layer chromatography was carried out on Sigma-Aldrich silica gel TLA aluminum foils with fluorescent indicator. Visualization was carried out under UV irradiation (254 and 365 nm) and by iodine staining. Temperatures are reported in °C. Mass spectra were performed on a LCQ (Finnigan-Mat) ion trap mass spectrometer (San Jose, CA) equipped with an electrospray ionization (ESI) source. The capillary temperature was set at 300 °C and the spray voltage at 3.5 kV. The fluid was nebulized using nitrogen as both the sheath gas and the auxiliary gas.²⁷

### Methyl ester formation (reaction a)

To a solution of Boc-amino acid in dried MeOH cooled to -15°C, thionyl chloride (2 eq.) was added dropwise. After stirring at -15°C for 30 min, the mixture was taken to r.t. in stirring overnight. Then the mixture was evaporated under reduced pressure to give the crude methyl ester derivative, which was used for the next reaction, without further purification. This protocol was applied to the preparation of HCl·H-Val-OMe and HCl·H-Leu-OMe.

### Coupling reaction (reaction b)

To a stirred solution of Lonidamine and HOBt (1.1 eq.) in DMF, EDC·HCl (1.1 eq.) and NMM (1.1 eq.) were added at 0°C, followed by an ice-cold suspension of HCl·H-L-*tert*-Leu-OMe and NMM (1.1 eq.) in DMF. After 10 min at 0°C, the reaction was stirred at r.t overnight. Later the reaction mixture was evaporated to dryness and the residue taken up in EtOAc. The organic phase was washed with 5% citric acid, saturated aqueous NaHCOs and NaCl, dried and evaporated to obtain the desired ester as crude compound.

### Saponification (reaction c)

To a solution of Lonidamine methyl ester in MeOH, NaOH 1N (6 eq.) was added under stirring at r.t. When the reaction was over, the solution was acidified with HCl 1N and extracted with EtOAc. The organic phase was washed with water, dried and evaporated to yield crude product.

### Amidation (reaction d))

Lonidamine, NMM (2.5 eq.) and isobutyl chloroformate (2.1 eq.) were added dropwise to a solution of the Boc-amino acid in THF and the mixture was continuously stirred at -20°C for 30 min. After that, NH₄OH solution was added to the reaction, stirring at -20°C for 30 min. The reaction was maintained for 24 h at r.t. The reaction mixture was evaporated to dryness and the residue extracted with EtOAc. The organic phase was washed with 5% citric acid, saturated aqueous NaHCOs and NaCl, dried and evaporated to obtain the desired amide derivative as crude compound.

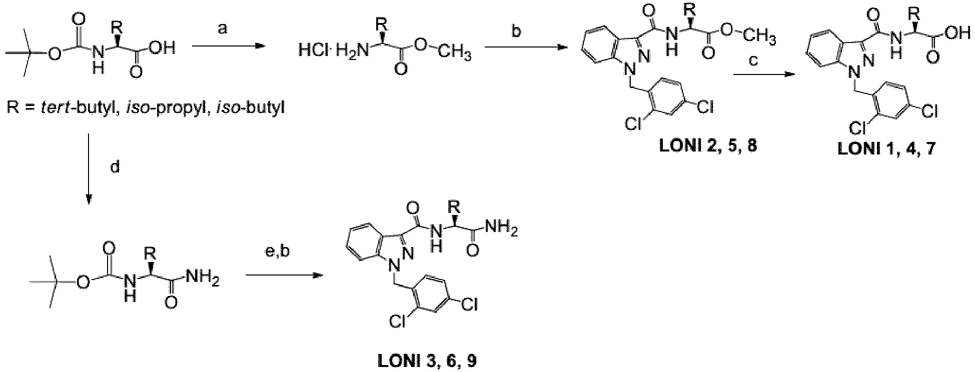

Wherein in the synthesis of Lonidamine based-compounds. a) MeOH, SOCl₂. b) Lonidamine, HOBt, EDC·HCl, NMM, DMF. c) NaOH 1N, in MeOH. d) NMM, isobutyl chloroformate, NH₄OH in THF, -20°C. e) TFA/DCM, 1h, r.t.

Lonidamine-L-tert-Leu-OH (LONI 1). Boc-tert-Leu-OH was transformed in its methyl ester derivative following the general procedure A. The so obtained product was coupled with lonidamine following the general procedure B, to give an intermediate product with was converted in LONI 1, following the general procedure C. Yield 30%. R*_{f}* =0.64 (EtOA/n-hexane 8:2); ¹H-NMR (DMSO-*d*₆) δ: 8.17 (d, 1H, H5'), 7.74 (d, 1H, H4), 7.70 (s, 1H, H3'), 7.59 (d, 1H, NH), 7.47 (t, 1H, H5), 7.28-7.37 (m, 2H, H6 and H6'), 6.84 (d, 1H, H7), 5.85 (s, 2H, H-1), 4.29-4.32 (m, 1H, CH^{α} L-*tert*-Leu), 0.98 (s, 9H, CH₃×3 L-*tert*-Leu). LRMS calcd.: 436.11, found: 437.15.

Lonidamine-L-tert-Leu-OMe (LONI 2): Boc-tert-Leu-OH was transformed in its methyl ester derivative following the general procedure A. The so obtained product was coupled with lonidamine following the general procedure B, to give LONI 2 as crude material. The residue was chromatographed on silica gel using EtOAc/ n-hexane = 20:80 as eluent. R*_{f}* =0.68 (EtOAc/n-hexane 30:70); Yield 25%. ¹H-NMR (DMSO-*d*₆) δ: 8.13 (d, 1H, H5'), 7.76 (d, 1H, H4), 7.70 (s, 1H, H3'), 7.65 (d, 1H, NH), 7.31-7.37 (m, 2H, H6 and H6'), 6.87 (d, 1H, H7), 5.85 (s, 2H, H-1), 4.43-4.47 (m, 1H, CH^{α} L-tert-Leu), 3.66 (s, 3H, OCH₃ *L-tert* Leu), 0.98 (s, 9H, CH₃×3 L-tert-Leu). LRMS calcd.: 448.12, found: 449.18.

Lonidamine-L-*tert*-Leu-NH₂ (LONI 3). Boc-L-*tert*-Leu-OH has been converted to its amide derivative following the general procedure D. The Boc-protection has been removed, then the intermediate compound has been reacted with Lonidamine, following the general procedure B, to give LONI 3. The residue was chromatographed on silica gel column using EtOAc/n-hexane from 20:80 to 40:60 as eluent. R*_{f}* =0.11 (EtOAc/n-hexane 3:7); Yield 90%. ¹H-NMR (DMSO-*d*₆) δ: 8.18 (d, 1H, H5'), 7.73 (d, 1H, H4), 7.71, 7.26 (brs, 2H, NH₂),7.69 (s, 1H, H3'), 7.54 (d, 1H, NH), 7.46 (t, 1H, H5), 7.30-7.36 (m, 2H, H6 and H6'), 5.84 (s, 2H, H-1), 4.41-4.45 (m, 1H, CH^{α} L-*tert*-Leu), 0.95 (s, 9H, CH₃×3 L-*tert*-Leu). LRMS calcd.: 435.35, found: 436.39.

Lonidamine-Val-OH (LONI 4). Boc-Val-OH was transformed in its methyl ester derivative following the general procedure A. The so obtained product was coupled with lonidamine following the general procedure B, to give an intermediate product which was converted in LONI 4 following the general procedure C. Yield 88%. R*_{f}* =0.71 (EtOAc); ¹H-NMR (DMSO-*d*₆) δ: 8.17 (d, 1H, H5'), 7.86 (d, 1H, H4), 7.75 (d, 1H, NH), 7.70 (s, 1H, H3'), 7.47 (t, 1H, H5), 7.28-7.36 (m, 2H, H6 and H6'), 6.79 (d, 1H, H7) 5.85 (s, 2H, H-1), 4.35-4.39 (m, 1H, CH^{α} Val), 2.20-2.22 (m, 1H, CH(CH₃)₂), 0.94 (t, 6H, CH₃×2 Val). LRMS calcd.: 420.10, found: 421.16.

Lonidamine-Val-OMe (LONI 5). Boc-Val-OH was transformed in its methyl ester derivative following the general procedure A. The so obtained product was coupled with lonidamine following the general procedure B to give LONI 5. The crude product was chromatographed on silica gel using EtOAc/ n-hexane from 20:80 to 30:70 as eluent. Yield 76%. R*_{f}* =0.67 (EtOAc/n-hexane 30:70); ¹H-NMR (DMSO-*d*₆) δ: 8.14-8.14-8.18 (m, 2H, H5' and H4), 7.74 (d, 1H, NH), 7.70 (s, 1H, H3'), 7.47 (t, 1H, H5), 7.28-7.37 (m, 2H, H6 and H6'), 6.77 (d, 1H, H7), 5.84 (s, 2H, H-1), 4.36-4.40 (m, 1H, CH^{α} Val), 3.65 (s, 3H, OCH₃ Val), 2.21-2.25 (m, 1H, CH(CH₃)₂), 0.90 (dd, 6H, CH₃×2 Val). LRMS: calcd. 434.11, found: 435.19.

Lonidamine-Val-NH₂ (LONI 6). Boc-Val-OH has been converted to its amide derivative following the general procedure D. The Boc-protection has been removed, then the intermediate compound has been reacted with Lonidamine, following the general procedure B, to give LONI 6. The residue was chromatographed on silica gel column using EtOAc/n-hexane = 80:20 as eluent. Yield 30%. R*_{f}* =0.67 (EtOAc/n-hexane 80:20); ¹H-NMR (DMSO-*d*₆) δ: 8.18 (d, 1H, H5'), 7.74 (d, 1H, H4), 7.68-7.71 (2H, m, NH and H3'), 7.22, 7.64 (brs, 2H, NH₂), 7.46 (t, 1H, H5), 7.27-7.36 (m, 2H, H6 and H6'), 6.79 (d, 1H, H7), 5.84 (s, 2H, H-1), 4.35-4.40 (m, 1H, CH^{α} Val), 2.03-2.09 (m, 1H, CH(CH₃)₂), 0.88 (dd, 6H, CH₃×2 Val). LRMS calcd.: 419.10, found: 420.13.

Lonidamine-L-Leu-OH (LONI 7). Boc-Leu-OH was transformed in its methyl ester derivative following the general procedure A. The so obtained product was coupled with lonidamine following the general procedure B. The desired product LONI 7 was prepared following the general procedure C starting from the intermediate product. Yield 93%. R*_{f}* =0.56 (EtOA); ¹H-NMR (DMSO-*d*₆) δ: 12.34 (1H, s, OH), 8.33 (d, 1H, H5'), 8.18 (d, 1H, H4), 7.74 (d, 1H, NH), 7.71 (s, 1H, H3'), 7.46 (t, 1H, H5), 7.27-7.35 (m, 2H, H6 and H6'), 6.73 (d, 1H, H7), 5.84 (s, 2H, H-1), 4.46-4.54 (m, 1H, CH^{α} Leu), 1.53-1.68 (m, 3H, CH₂CH(CH₃)₂, CH(CH₃)₂), 0.86-0.82 (dd, 6H, CH₃×2 Leu). LRMS calcd.: 434.11, found: 435.19.

Lonidamine-L-Leu-OMe (LONI 8). Boc-Leu-OH was transformed in its methyl ester derivative following the general procedure A. The so obtained product was coupled with lonidamine following the general procedure B, to give LONI 8. Yield 95%. R*_{f}* =0.61 (EtOAc/n-hexane 70:30); ¹H-NMR (DMSO-*d*₆) δ: 8.57 (d, 1H, H5'), 8.17 (d, 1H, H4), 7.74 (d, 1H, NH), 7.71 (s, 1H, H3'), 7.46 (t, 1H, H5), 7.27-7.36 (m, 2H, H6 and H6'), 6.71 (d, 1H, H7), 5.84 (s, 2H, H-1), 4.52-4.60 (m, 1H, CH^{α} Leu), 3.62 (s, 3H, OCH₃ Leu), 1.80-1.88 (m, 1H, , CH(CH₃)₂), 1.54-1.64 (m, 2H, CH₂CH(CH₃)₂), 0.87 (dd, 6H, CH₃×2 Leu). LRMS calcd.: 448.12, found: 449.15.

Lonidamine-L-Leu-NH₂ (LONI 9). Boc-L-Leu-OH has been converted to its amide derivative following the general procedure D. The Boc-protection has been removed, then the intermediate compound has been reacted with Lonidamine, following the general procedure B, to give LONI 9. The residue was chromatographed on silica gel using EtOAc/ n-hexane = 60:40 as eluent. Yield 83%. R*_{f}* =0.58 (EtOAc/n-hexane 7:3); ¹H-NMR (DMSO-*d*₆) δ: 8.19 (d, 1H, H5'), 7.93 (d, 1H, NH), 7.73 (d, 1H, H4), 7.69 (s, 1H, H3'), 7.09, 7.52 (brs, 2H, NH₂) 7.46 (t, 1H, H5), 7.27-7.35 (m, 2H, H6 and H6'), 6.77 (d, 1H, H7), 5.82 (s, 2H, H-1), 4.50-4.52 (m, 1H, CH^{α} Leu), 1.55-1.63 (m, 3H, CH₂CH(CH₃)₂ and CH(CH₃)₂), 0.88 (dd, 6H, CH₃×2 Leu). LRMS calcd.: 433.11, found: 433.14.

### Rat brain membrane preparation

Wistar rats were locally bred and handled according to the European Communities Council Directives (86/609/ECC) and the Hungarian Act for the Protection of Animals in Research (XXVlll.tv. Section 32). Crude membrane fractions were prepared from the brain without cerebellum. Brains were quickly removed from the euthanized rats and directly put in ice-cold 50 mM Tris-HCl buffer (pH 7.4). The collected tissue was then homogenized in 30 volumes (v/w) of ice-cold buffer with a Braun Teflon-glass homogenizer at the highest rpm. The homogenate was centrifuged at 20,000 g for 25 min and the resulting pellet was suspended in the same volume of cold buffer followed by incubation at 37°C for 30 min to remove endogenous ligands. Centrifugation was then repeated. The final pellets were taken up in five volumes of 50 mM Tris-HCl (pH 7.4) buffer containing 0.32 M sucrose and stored at -80°C. Prior to the experiment, aliquots were thawed and centrifuged at 20,000 g for 25 min and the were resuspended in 50 mM Tris-HCl (pH 7.4), homogenized with a Dounce in followed by the determination of the protein concentration by the method of Bradford.

### Competition binding experiments

Binding experiments were performed at 30oC for 1 h in 50 mM Tris-HCl buffer pH 7.4 containing 2.5 mM EGTA, 5 mM MgCl2 and 0.5 mg/mL fatty acid free BSA in plastic tubes in a total assay volume of 1 mL that contained 0.3-0.5 mg/mL membrane protein.

Competition binding experiments were carried out by incubating rat brain membranes with 1.5 nM of [³H]WIN55212-2 in the presence of increasing concentrations (10⁻¹¹-10⁻⁵ M) of various competing unlabeled ligands. Non-specific binding was determined in the presence of 10 µM WIN 55212-2. The incubation was terminated by diluting the samples with ice-cold wash buffer (50 mM Tris-HCl, 2.5 mM EGTA, 5 mM MgCl₂, 0.5% fatty acid free BSA, pH 7.4), followed by repeated washing and rapid filtration through Whatman GF/B glass fiber filters (Whatman Ltd, Maidstone, England) presoaked with 0.1% polyethyleneimine (30 min before the filtration). Filtration was performed with a 24-well Brandel Cell Harvester (Gaithersburg, MD, USA). Filters were air-dried and immersed into Ultima Gold MV scintillation cocktail and then radioactivity was measured with a TRI-CARB 2100TR liquid scintillation analyzer (Packard).

### Ligand stimulated [³⁵S]GTPγS binding assay

Rat brain membranes (30 µg protein/tube) prepared as described above, were incubated with 0.05 nM [³⁵S]GTPγS (PerkinElmer) and 10⁻¹⁰-10⁻⁵ M unlabeled ligands in the presence of 30 µM GDP, 100 mM NaCl, 3 mM MgCl₂ and 1 mM EGTA in 50 mM Tris-HCl buffer (pH 7.4) for 60 min at 30°C. Basal [³⁵S]GTPγS binding was measured in the absence of ligands and set as 100%. Nonspecific binding was determined by the addition of 10 µM unlabeled GTPγS and subtracted from total binding. Incubation, filtration and radioactivity measurement of the samples were carried out as described above.

### Data analysis

Results are expressed as means ± S.E.M. of at least three independent experiments each performed in duplicate. In competition binding studies, the inhibitory constants (Kᵢ) were calculated from the inflection points of the displacement curves using non-linear least-square curve fitting and the Cheng- Prusoff equation. All data and curves were analyzed by GraphPad Prism 5.0 (San Diego, CA, USA). In [³⁵S]GTPγS binding studies, data were expressed as the percentage stimulation of the specific [³⁵S]GTPγS binding over the basal activity and are given as means ± S.E.M. Each experiment was performed in triplicate and analyzed with sigmoid dose-response curve fitting to obtain potency (EC₅₀) and efficacy (Eₘₐₓ) values. Statistical comparison was done by analysis of variance (one-way ANOVA) followed by the Bonferroni multiple comparison test of GraphPad Prism 5.0 (San Diego, CA, USA), P< 0.05 was chosen to indicate significant differences.

### Results

Compounds (LONI 1-9) were synthesized in good to excellent yields by standard solution phase peptide synthesis (SPPS) via EDC/HOBt-mediated condensation in the presence of NMM as base, using Boc strategy following well-established procedures; N-Boc deprotection was carried out by TFA treatment. The Lonidamine derivatives LONI B (Lonidamine-NH₂) and LONI C (Lonidamine-OCH₃) were also prepared according to the general procedures D and A, respectively, for the biological assays. Compounds LONI 1-9 are reported in the following table 1. LONI 3, 6 and 9 are for reference only.

**Table 1**

| **COMPOUN D** | **SEQUENCE** | **MOLECULE** |
|---|---|---|
| **LONI 1** | Chemical Formula: C₂₁H₂₁Cl₂N₃O₃ | 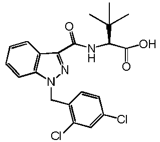 |
| | Molecular Weight: 434,3157 g/mol | |
| | (*S*)-2-(1-(2,4-dichlorobenzyl)-*1H*-indazole-3-carboxamido)-3,3-dimethylbutanoic acid | |
| **LONI 2** | Chemical Formula: C₂₂H₂₃Cl₂N₃O₃ | 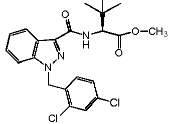 |
| | Molecular Weight: 448,3423 g/mol | |
| | (*S*)-methyl 2-(1-(2,4-dichlorobenzyl)-1*H*-indazole-3-carboxamido)-3,3-dimethylbutanoate | |
| **LONI 3** | Chemical Formula: C₂₁H₂₂Cl₂N₄O₂ | 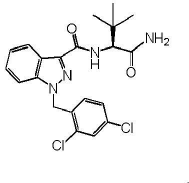 |
| | Molecular Weight: 433,3310 g/mol | |
| | (*S*)-*N*-(1-amino-3,3-dimethyl-1-oxobutan-2-yl)-1-(2,4-dichlorobenzyl)-1*H*-indazole-3 carboxamide | |
| **LONI 4** | Chemical Formula: C₂₀H₁₉Cl₂N₃O₃ | 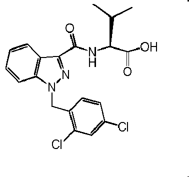 |
| | Molecular Weight: 420,2892 g/mol | |
| | (*S*)-2-(1-(2,4-dichlorobenzyl)-1*H*-indazole-3-carboxamido)-3-methylbutanoic aci | |
| **LONI 5** | Chemical Formula: C₂₁H₂₁Cl₂N₃O₃ | 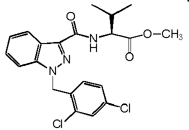 |
| | Molecular Weight: 434,3157 g/mol | |
| | (*S*)-methyl 2-(1-(2,4-dichlorobenzyl)-1*H*-indazole-3-carboxamido)-3-methylbutanoate | |
| **LONI 6** | Chemical Formula: C₂₀H₂₀Cl₂N₄O₂ | 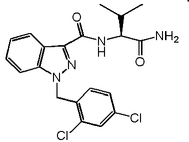 |
| | Molecular Weight: 419,3044 g/mol | |
| | (*S*)-*N*-(1-amino-3-methyl-1-oxobutan-2-yl)-1-(2,4-dichlorobenzyl)-1*H*-indazole-3-carboxamide | |
| **LONI 7** | Chemical Formula: C₂₁H₂₁Cl₂N₃O₃ | 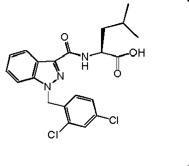 |
| | Molecular Weight: 434,3157 g/mol | |
| | (*S*)-2-(1-(2,4-dichlorobenzyl)-1*H*-indazole-3-carboxamido)-4-methylpentanoic acid | |
| **LONI 8** | Chemical Formula: C₂₂H₂₃Cl₂N₃O₃ | 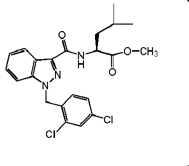 |
| | Molecular Weight: 448,3423 g/mol | |
| | (*S*)-methyl 2-(1-(2,4-dichlorobenzyl)-1*H*-indazole-3-carboxamido)-4-methylpentanoa | |
| **LONI 9** | Chemical Formula: C₂₁H₂₂Cl₂N₄O₂ | 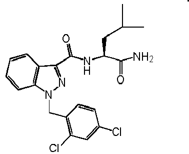 |
| | Molecular Weight: 433,3310 g/mol | |
| | (*S*)-*N*-(1-amino-4-methyl-1-oxopentan-2-yl)-1-(2,4-dichlorobenzyl)-1*H*-indazole-3-carboxamide | |

Competition binding experiments were performed to compare the ability of the parent ligand Lonidamine (**LONI A)** and its derivatives (**LONI B, LONI C, LONI 1-9)** to inhibit the binding of [³H]WIN55212-2 in rat brain membrane homogenate. It was found that neither **LONI A** nor its derivatives **LONI B and LONI C** could displace [³H]WIN55212-2. Overall the Lonidamine analogues showed a wide range of binding to CB receptors from subnanomolar (Kᵢ= 0.08 nM) to low nanomolar (Kᵢ= 88 nM) affinity. **LONI 1, LONI 2, LONI 4** and **LONI 5** showed the highest affinity for CB receptors. In our experimental model, the investigated ligands competed for [³H]WIN55212-2 binding sites with the following order of potency: **LONI 1** > **LONI 5** > **LONI 4** > **LONI 2** > **LONI 3, LONI 6** > **LONI 7** > **LONI 8** > **LONI 9** (Figure 1).

In order to determine whether the parent ligand and its derivatives are able to stimulate the CB receptor-associated G-proteins, these compounds were subjected to ligand-stimulated [³⁵S]GTPγS binding assays in rat brain membrane homogenate. In these in vitro functional assays **LONI A** didn't show G-protein stimulation. The compounds **LONI B and LONI C** were found to be weak inverse agonist, but the G-protein stimulation was not CB receptors-mediated. **LONI 2, LONI 5** and **LONI 8** efficiently stimulated the G-proteins, demonstrating high potency (EC₅₀= 8-33 nM) and high stimulatory activity (Eₘₐₓ= 122-143%). **LONI 3, LONI 6** and **LONI 9** were equipotent partial agonists (Eₘₐₓ= 120-139%) with lower potency (EC₅₀= 126-317 nM), while the introduction of the carboxyl function in **LONI 1, LONI 4** and **LONI 7** resulted in weak inverse agonist activity with similar parameters: the maximum efficacy was between 82-86%, and their potency were between 1.8-3.2 µM, results are shown in figure 2 and in the following table 2, wherein Kᵢ values were calculated as Kᵢ= EC₅₀/(1 + [ligand]/K_{d}), where K_{d}([³H]WIN55212-2)= 10.1 nM. The corresponding displacement curves are shown in Figure 1. The Eₘₐₓ and EC₅₀ values were calculated from the dose-response curves of Figure 2. *Mean of three independent measurements (S.E.M. values are in the range of 5-12%).

**Table 2**

| **Compounds** | **Kᵢ (nM)*** | **Eₘₐₓ (%) ±SEM** | **EC₅₀ (nM)** |
|---|---|---|---|
| JWH-018 | 3.5 | 163 ± 5.2 | 16 |
| **LONI A** | - | - | - |
| **LONI B** | - | 70 ± 8.7 | 2784 |
| **LONI C** | - | 69 ± 2.6 | 3159 |
| **LONI 1** | 0.08 | 84 ± 6.6 | 2803 |
| **LONI 2** | 3.1 | 143 ±5.7 | 8.4 |
| **LONI 3** | 17 | 139 ± 4.5 | 126 |
| **LONI 4** | 2.6 | 82 ± 10.6 | 3293 |
| **LONI 5** | 1.8 | 140 ± 3.7 | 33 |
| **LONI 6** | 17 | 135 ± 4.9 | 273 |
| **LONI 7** | 43 | 86 ± 9.3 | 1870 |
| **LONI 8** | 63 | 122 ± 4.6 | 19 |
| **LONI 9** | 88 | 120 ± 5.9 | 317 |

In the next step the compounds were evaluated for CB1/CB2 receptor selectivity by [³⁵S]GTPγS binding assays. The G-protein stimulatory effect of compounds **LONI 1-LONI 9** (10 µM) was reduced by the CB1 receptor antagonist/inverse agonist rimonabant (10 µM) and by the CB2 receptor antagonist/inverse agonist AM630 (10 µM), and a 3-6-fold selectivity for CB1 receptor was observed. **LONI 3** and **LONI 9** exhibited 6-fold selectivity for CB1 receptor, while **LONI 4** demonstrated only 3-fold selectivity for CB1 receptor. The other compounds have 4-5-fold selectivity for the CB1 receptor, results are shown in figure 3.

In summary Lonidamine-based compounds exhibited a broad range of activities. **LONI 1, 2, 4** and **5** showed the highest affinity for CB receptors with sub- and low nanomolar inhibitory constant (Kᵢ) values; **LONI 2, 5** and **8** efficiently stimulated the G-proteins, demonstrating low potency and high stimulatory activity. **LONI 3, 6** and **9** acted as partial agonists with high potency, while the introduction of the carboxyl function in **LONI 1, 4** and **7** resulted in inverse agonists with very similar parameters. **LONI 3** and **LONI 9** exhibited the highest (6-fold) selectivity for CB1 receptor, while **LONI 4** demonstrated the lowest, 3-fold selectivity for CB1 receptor. The other compounds have 4-5-fold selectivity for the CB1 receptor. Compounds with antagonist activity could potentially act as an anorectic antiobesity drug, while Lonidamine-based compounds with agonist activity could be used to treat chemotherapy-related nausea and vomiting or to treat loss of appetite and weight in AIDS patients and could be involved in the treatment of pain.

## Claims

1. Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate

2. Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate
for use as medicaments.

3. Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate
for use as modulator or antagonist or inverse agonist of cannabinoids receptor.

4. Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate
for use as anorectic agent.

5. Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate
for use for the treatment of eating diseases.

6. Compounds selected form the group consisting of:
(*S*)-2-(2,4-dichlorobenzyl)-*1H*-indazole-3-carboxamido)-3,3-dimethylbutanoic acid
(*S*)-methyl 2-(1-(2,4-dichlorobenzyl)-*1H*-indazole-3-carboxamido)-3,3-dimethylbutanoate
(*S*)-2-(2,4-dichlorobenzyl)-*1H*-indazole-3-carboxamido)-3-methylbutanoic acid
(*S*)-methyl 2-(1-(2,4-dichlorobenzyl)-*1H*-indazole-3-carboxamido)-3-methylbutanoate
(*S*)-2-(1-(2,4-dichlorobenzyl)-*1H*-indazole-3-carboxamido)-4-methylpentanoic acid
(*S*)-methyl 2-(1-(2,4-dichlorobenzyl)-*1H*-indazole-3-carboxamido)-4-methylpentanoate
in combination with at least one chemoterapeutic agent for the treatment of cancer.

## Patentansprüche

1. Verbindungen, ausgewählt aus der Gruppe bestehend aus:
(*S*)-2-(2,4-Dichlorbenzyl)-*1H*-indazol-3-carboxamido*)*-3,3-dimethylbutansäure
(*S*)-Methyl-2-(1-(2,4-dichlorbenzyl)-*1H*-indazol-3-carboxamido)-3,3-dimethylbutanoat
(S)-2-(2,4-Dichlorbenzyl)-*1H*-indazol-3-carboxamido)-3-methylbutansäure
(*S*)-Methyl- 2-(1-(2,4-dichlorbenzyl)-*1H*-indazol-3-carboxamido)-3-methylbutanoat
(*S*)-2-(1-(2,4-Dichlorbenzyl)-*1H*-indazol-3-carboxamido )-4-methylpentansäure
(*S*)-Methyl-2-(1-(2,4-dichlorbenzyl)-*1H*-indazol-3-carboxamido)-4-methylpentanoat

2. Verbindungen, ausgewählt aus der Gruppe bestehend aus:
(*S*)-2-(2,4-Dichlorbenzyl)-*1H*-indazol-3-carboxamido)-3,3-dimethylbutansäure
(*S*)-Methyl-2-(1-(2,4-dichlorbenzyl)-*1H*-indazol-3-carboxamido)-3,3-dimethylbutanoat
(*S*)-2-(2,4-Dichlorbenzyl)-*1H*-indazol-3-carboxamido)-3-methylbutansäure
(*S*)-Methyl-2-(1-(2,4-dichlorbenzyl)-*1H*-indazol-3-carboxamido)-3-methylbutanoat
(*S*)-2-(1-(2,4-Dichlorbenzyl)-*1H*-indazol-3-carboxamido)-4-methylpentansäure
(*S*)-Methyl-2-(1-(2,4-dichlorbenzyl)-*1H*-indazol-3-carboxamido)-4-methylpentanoat
zur Verwendung als Medikamente.

3. Verbindungen, ausgewählt aus der Gruppe bestehend aus:
(*S*)-2-(2,4-Dichlorbenzyl)-*1H*-indazol-3-carboxamido)-3,3-dimethylbutansäure
(*S*)-Methyl-2-(1-(2,4-dichlorbenzyl)-*1H*-indazol-3-carboxamido)-3,3-dimethylbutanoat
(*S*)-2-(2,4-Dichlorbenzyl)-*1H*-indazol-3-carboxamido)-3-methylbutansäure
(*S*)-Methyl-2-(1-(2,4-dichlorbenzyl)-*1H*-indazol-3-carboxam ido)-3-methylbutanoat
(*S*)-2-(1-(2,4-Dichlorbenzyl)-*1H*-indazol-3-carboxam ido)-4-methylpentansäure
(*S*)-Methyl-2-(1-(2,4-dichlorbenzyl)-*1H*-indazol-3-carboxam ido)-4-methylpentanoat
zur Verwendung als Modulator oder Antagonist oder inverser Agonist für Cannabinoid-Rezeptoren.

4. Verbindungen, ausgewählt aus der Gruppe bestehend aus:
(*S*)-2-(2,4-Dichlorbenzyl)-*1H*-indazol-3-carboxamido)-3,3-dimethylbutansäure
(*S*)-Methyl-2-(1-(2,4-dichlorbenzyl)-*1H*-indazol-3-carboxamido)-3,3-dimethylbutanoat
*(S)*-2-(2,4-Dichlorbenzyl)-*1H*-indazol-3-carboxam ido)-3-methylbutansäure
(*S*)-Methyl-2-(1-(2,4-dichlorbenzyl)-*1H*-indazol-3-carboxamido)-3-methylbutanoat
(*S*)-2-(1-(2,4-Dichlorbenzyl)-*1H*-indazol-3-carboxamido)-4-methylpentansäure
(*S*)-Methyl 2-(1-(2,4-dichlorbenzyl)-*1H*-indazol-3-carboxamido)-4-methylpentanoat
zur Verwendung als Anorekatikum.

5. Verbindungen, ausgewählt aus der Gruppe bestehend aus:
(*S*)-2-(2,4-Dichlorbenzyl)-*1H*-indazol-3-carboxamido)-3,3-dimethylbutansäure
(*S*)-Methyl-2-(1-(2,4-dichlorbenzyl)-*1H*-indazol-3-carboxamido)-3,3-dimethylbutanoat
*(S)*-2-(2,4-Dichlorbenzyl)-*1H*-indazol-3-carboxamido)-3-methylbutansäure
(*S*)-Methyl-2-(1-(2,4-dichlorbenzyl)-*1H*-indazol-3-carboxamido)-3-methylbutanoat
(*S*)-2-(1-(2,4-Dichlorbenzyl)-*1H*-indazol-3-carboxamido)-4-methylpentansäure
(*S*)-Methyl-2-(1-(2,4-dichlorbenzyl)-*1H*-indazol-3-carboxamido)-4-methylpentanoat
zur Verwendung für die Behandlung von Essstörungen.

6. Verbindungen, ausgewählt aus der Gruppe bestehend aus:
*(S)*-2-(2,4-Dichlorbenzyl)-*1H*-indazol-3-carboxamido)-3,3-dimethylbutansäure
(*S*)-Methyl-2-(1-(2,4-dichlorbenzyl)-*1H*-indazol-3-carboxamido)-3,3-dimethylbutanoat
*(S)*-2-(2,4-Dichlorbenzyl)-*1H*-indazol-3-carboxamido)-3-methylbutansäure
(*S*)-Methyl-2-(1-(2,4-dichlorbenzyl)-*1H*-indazol-3-carboxamido)-3-methylbutanoat
(*S*)-2-(1-(2,4-Dichlorbenzyl)-*1H*-indazol-3-carboxamido)-4-methylpentansäure
(S)-Methy-2-(1-(2,4-dichlorbenzyl)-*1H*-indazol-3-carboxamido)-4-methylpentanoat
in Kombination mit mindestens einem Chemotherapeutikum für die Behandlung von Krebs.

## Revendications

1. Composés choisis dans le groupe constitué de :
l'acide (S)-2-(2,4-dichlorobenzyl)-1H-indazole-3-carboxamido)-3,3-diméthylbutanoïque
le 2-(1-(2,4-dichlorobenzyl)-1H-indazole-3-carboxamido)-3,3-diméthylbutanoate de (S)-méthyle
l'acide (S)-2-(2,4-dichlorobenzyl)-1H-indazole-3-carboxamido)-3-méthylbutanoïque
le 2-(1-(2,4-dichlorobenzyl)-1H-indazole-3-carboxamido)-3-méthylbutanoate de (S)-méthyle
l'acide (S)-2-(1-(2,4-dichlorobenzyl)-1H-indazole-3-carboxamido)-4-méthylpentanoïque
le 2-(1-(2,4-dichlorobenzyl)-1H-indazole-3-carboxamido)-4-méthylpentanoate de (S)-méthyle.

2. Composés choisis dans le groupe constitué de :
l'acide (S)-2-(2,4-dichlorobenzyl)-1H-indazole-3-carboxamido)-3,3-diméthylbutanoïque
le 2-(1-(2,4-dichlorobenzyl)-1H-indazole-3-carboxamido)-3,3-diméthylbutanoate de (S)-méthyle
l'acide (S)-2-(2,4-dichlorobenzyl)-1H-indazole-3-carboxamido)-3-méthylbutanoïque
le 2-(1-(2,4-dichlorobenzyl)-1H-indazole-3-carboxamido)-3-méthylbutanoate de (S)-méthyle
l'acide (S)-2-(1-(2,4-dichlorobenzyl)-1H-indazole-3-carboxamido)-4-méthylpentanoïque
le 2-(1-(2,4-dichlorobenzyl)-1H-indazole-3-carboxamido)-4-méthylpentanoate de (S)-méthyle
pour une utilisation en tant que médicaments.

3. Composés choisis dans le groupe constitué de :
l'acide (S)-2-(2,4-dichlorobenzyl)-1H-indazole-3-carboxamido)-3,3-diméthylbutanoïque
le 2-(1-(2,4-dichlorobenzyl)-1H-indazole-3-carboxamido)-3,3-diméthylbutanoate de (S)-méthyle
l'acide (S)-2-(2,4-dichlorobenzyl)-1H-indazole-3-carboxamido)-3-méthylbutanoïque
le 2-(1-(2,4-dichlorobenzyl)-1H-indazole-3-carboxamido)-3-méthylbutanoate de (S)-méthyle
l'acide (S)-2-(1-(2,4-dichlorobenzyl)-1H-indazole-3-carboxamido)-4-méthylpentanoïque
le 2-(1-(2,4-dichlorobenzyl)-1H-indazole-3-carboxamido)-4-méthylpentanoate de (S)-méthyle
pour une utilisation en tant que modulateur ou antagoniste ou agoniste inverse d'un récepteur des cannabinoïdes.

4. Composés choisis dans le groupe constitué de :
l'acide (S)-2-(2,4-dichlorobenzyl)-1H-indazole-3-carboxamido)-3,3-diméthylbutanoïque
le 2-(1-(2,4-dichlorobenzyl)-1H-indazole-3-carboxamido)-3,3-diméthylbutanoate de (S)-méthyle
l'acide (S)-2-(2,4-dichlorobenzyl)-1H-indazole-3-carboxamido)-3-méthylbutanoïque
le 2-(1-(2,4-dichlorobenzyl)-1H-indazole-3-carboxamido)-3-méthylbutanoate de (S)-méthyle
l'acide (S)-2-(1-(2,4-dichlorobenzyl)-1H-indazole-3-carboxamido)-4-méthylpentanoïque
le 2-(1-(2,4-dichlorobenzyl)-1H-indazole-3-carboxamido)-4-méthylpentanoate de (S)-méthyle
pour une utilisation en tant qu'agent anorexigène.

5. Composés choisis dans le groupe constitué de :
l'acide (S)-2-(2,4-dichlorobenzyl)-1H-indazole-3-carboxamido)-3,3-diméthylbutanoïque
le 2-(1-(2,4-dichlorobenzyl)-1H-indazole-3-carboxamido)-3,3-diméthylbutanoate de (S)-méthyle
l'acide (S)-2-(2,4-dichlorobenzyl)-1H-indazole-3-carboxamido)-3-méthylbutanoïque
le 2-(1-(2,4-dichlorobenzyl)-1H-indazole-3-carboxamido)-3-méthylbutanoate de (S)-méthyle
l'acide (S)-2-(1-(2,4-dichlorobenzyl)-1H-indazole-3-carboxamido)-4-méthylpentanoïque
le 2-(1-(2,4-dichlorobenzyl)-1H-indazole-3-carboxamido)-4-méthylpentanoate de (S)-méthyle
pour une utilisation pour le traitement des troubles alimentaires.

6. Composés choisis dans le groupe constitué de :
l'acide (S)-2-(2,4-dichlorobenzyl)-1H-indazole-3-carboxamido)-3,3-diméthylbutanoïque
le 2-(1-(2,4-dichlorobenzyl)-1H-indazole-3-carboxamido)-3,3-diméthylbutanoate de (S)-méthyle
l'acide (S)-2-(2,4-dichlorobenzyl)-1H-indazole-3-carboxamido)-3-méthylbutanoïque
le 2-(1-(2,4-dichlorobenzyl)-1H-indazole-3-carboxamido)-3-méthylbutanoate de (S)-méthyle
l'acide (S)-2-(1-(2,4-dichlorobenzyl)-1H-indazole-3-carboxamido)-4-méthylpentanoïque
le 2-(1-(2,4-dichlorobenzyl)-1H-indazole-3-carboxamido)-4-méthylpentanoate de (S)-méthyle
en association avec au moins un agent chimiothérapeutique pour le traitement d'un cancer.
